# EUROPEAN PATENT APPLICATION

(11) **EP 3 422 357 A1**
(43) Date of publication of application: **02.01.2019**
(21) Application number: 18179294.6
(22) Date of filing: 22.06.2018
(51) Int. Cl.: G16H 50/20, G16H 40/63, G16H 40/67

(54) **AERIAL VEHICLE MEDICAL MANAGEMENT SYSTEM**

(30) Priority: 27.06.2017 US 201715634021
(71) Applicant: GE Aviation Systems LLC, Grand Rapids, MI 49512-1991 (US)
(72) Inventor: BOLLING, Randy E., Pinellas Park, FL Florida 33782 (US)
(74) Representative: Williams, Andrew Richard

(57) **Abstract**

One example aspect of the present disclosure relates to a method for assessing a health of a passenger of an aerial vehicle 102. The method can include receiving at least one signal indicative of a state of the passenger from one or more sensors of a chair in the aerial vehicle 102. The method can include determining a health state of the passenger based on the at least one received signal. The method can include determining if the aerial vehicle 102 should land based on the determined health state of the passenger. When a determination is made that the aerial vehicle 102 should land based on the determined health state of the passenger, the method can include identifying a nearest airport. When a determination is made that the aerial vehicle 102 should land based on the determined health state of the passenger, the method can include transmitting a request to land to the identified airport.

## Description

### FIELD

The present subject matter relates generally to an aerial vehicle.

### BACKGROUND

When a passenger or a crewmember on a flight of an aerial vehicle experiences a health scare, there may not be any good options on how to handle the incident. Landing an aerial vehicle prematurely can cost tens of thousands of dollars and causes inconvenience to other passengers. Therefore, landing an aerial vehicle prematurely because of a health scare that did not necessitate a premature landing should be avoided. However, airlines typically err on the side of caution, as a death of a passenger that could have been prevented by a premature landing is to be avoided at all costs.

### BRIEF DESCRIPTION

Aspects and advantages of aspects of the present disclosure will be set forth in part in the following description, or may be learned from the description, or may be learned through practice of the examples disclosed herein.

One example aspect of the present disclosure relates to a method for assessing a health of a passenger of an aerial vehicle. The method can include receiving at least one signal indicative of a state of the passenger from one or more sensors of a chair in the aerial vehicle. The method can include determining a health state of the passenger based on the at least one received signal. The method can include determining if the aerial vehicle should land based on the determined health state of the passenger. When a determination is made that the aerial vehicle should land based on the determined health state of the passenger, the method can include identifying a nearest airport. When a determination is made that the aerial vehicle should land based on the determined health state of the passenger, the method can include transmitting a request to land to the identified airport.

Another example aspect of the present disclosure relates to a system for assessing a health of a passenger of an aerial vehicle. The system can include a memory device. The system can include one or more processors. The one or more processors can be configured to receive at least one signal indicative of a state of the passenger from one or more sensors of a chair in the aerial vehicle. The one or more processors can be configured to determine a health state of the passenger based on the at least one received signal. The one or more processors can be configured to determine if the aerial vehicle should land based on the determined health state of the passenger. When a determination is made that the aerial vehicle should land based on the determined health state of the passenger, the one or more processors can be configured to adjust a flight plan.

Another example aspect of the present disclosure relates to an aerial vehicle capable of autonomous flight. The aerial vehicle can include a memory device. The aerial vehicle can include one or more processors. The one or more processors can be configured to receive at least one signal indicative of a state of the passenger from one or more sensors of a chair in the aerial vehicle. The one or more processors can be configured to determine a health state of the passenger based on the at least one received signal. The one or more processors can be configured to determine if the aerial vehicle should take action based on the determined health state of the passenger. When a determination is made that the aerial vehicle should take action based on the determined health state of the passenger, the one or more processors can be configured to send a signal to a control system to take an action.

Other example aspects of the present disclosure are directed to systems, methods, aerial vehicles, avionics systems, devices, non-transitory computer-readable media for assessing a health of a passenger of an aerial vehicle. Variations and modifications can be made to these example aspects of the present disclosure.

These and other features, aspects and advantages of various examples will become better understood with reference to the following description and appended claims. The accompanying drawings, which are incorporated in and constitute a part of this specification, illustrate aspects of the present disclosure and, together with the description, serve to explain the related principles.

### BRIEF DESCRIPTION OF THE DRAWINGS

Detailed discussion of embodiments directed to one of ordinary skill in the art are set forth in the specification, which makes reference to the appended figures, in which:
FIG. 1 depicts an aerial vehicle according to example embodiments of the present disclosure;
FIG. 2 depicts a block diagram of a sick chair according to example embodiments of the present disclosure;
FIG. 3 depicts a flow diagram of an example method according to example embodiments of the present disclosure;
FIG. 4 depicts a control system for implementing one or more aspects according to example embodiments of the present disclosure; and
FIG. 5 depicts example vehicles according to example embodiments of the present disclosure.

### DETAILED DESCRIPTION

Reference now will be made in detail to embodiments, one or more examples of which are illustrated in the drawings. Each example is provided by way of explanation of the embodiments, not limitation of the embodiments. In fact, it will be apparent to those skilled in the art that various modifications and variations can be made in the present disclosure without departing from the scope or spirit of the invention. For instance, features illustrated or described as part of one embodiment can be used with another embodiment to yield a still further embodiment. Thus, it is intended that the present disclosure covers such modifications and variations as come within the scope of the appended claims and their equivalents.

As used in the specification and the appended claims, the singular forms "a," "an," and "the" include plural referents unless the context clearly dictates otherwise. The use of the term "about" in conjunction with a numerical value refers to within 25% of the stated amount.

An aerial vehicle can include a sick chair. In an embodiment, the sick chair can be vacant in the absence of a passenger having a health scare. In an embodiment, the sick chair can be used as a normal passenger seat in the absence of a passenger having a health scare. In an embodiment, the sick chair can be designated for crew in the absence of a passenger having a health scare. When a passenger has a health scare that might necessitate a premature landing, the passenger can first be moved to the sick chair for evaluation.

In an embodiment, the sick chair can include a camera to detect a dilation of eyes and/or color of the eyes and/or skin of the passenger. In an embodiment, the sick chair can include a mask to detect oxygen and/or alcohol in the breath of the passenger. In an embodiment, the sick chair can include a thermometer for detecting a temperature of the passenger. In an embodiment, the sick chair can include scales to detect a weight of the passenger. In an embodiment, the sick chair can include a ruler and/or tape measure to detect a height of the passenger. In an embodiment, the sick chair can include a stethoscope. In an embodiment, the sick chair can include one or more cuffs to detect a blood pressure of the passenger. In an embodiment, the sick chair can include scissors for cutting clothing of the passenger. In an embodiment, the sick chair can include resuscitation tape. In an embodiment, the sick chair can include guidance for appropriate pediatric and adult equipment sizing and medication dosing. In an embodiment, the sick chair can include a pulse oximeter with adult and pediatric probes. In an embodiment, the sick chair can include a camera to examine an ear, nose, and/or throat of the passenger. In an embodiment, the sick chair can include a defibrillator. In an embodiment, the sick chair can include one or more tools for performing an electrocardiogram (EKG) and/or an echocardiogram (ECG), such as a transducer and/or machine. In an embodiment, the sick chair can include tools for testing for one or more viruses and/or bacteria, such as a litmus test or a scanner. In an embodiment, the sick chair can include an ultrasonic transducer and/or machine. In an embodiment, the sick chair can include a blood glucose meter and a pin prick. In an embodiment, the sick chair can include one or more spirometers for measuring a ventilation obstruction and/or a ventilation restriction associated with the passenger. In an embodiment, the sick chair can include a variety of tests, such as a strep throat test, a pregnancy test, etc. In an embodiment, the sick chair can include a privacy screen.

One or more processors associated with the sick chair can transmit one of more signals indicative of detected passenger information to a ground system. Someone at the ground system, such as a doctor, can interpret the signals indicative of the detected passenger information. The one or more processors associated with the sick chair can receive a recommendation from the ground system. In an embodiment, a recommendation can be made by the one or more processors associated with the sick chair by comparing the detected passenger information with one or more threshold values. The recommendation can include a recommendation to immediately land the aerial vehicle and get the passenger off the aerial vehicle. The recommendation can include a regimen for the passenger for a duration of a flight, such as recommended medicine, recommended increase water intake, recommended reduce alcohol intake, etc.

In this way, the systems and methods according to example aspects of the present disclosure can have a number of technical effects and benefits. For instance, example aspects of the present disclosure have a technical effect of reducing computational resources needed to determine if an aerial vehicle should land to accommodate a passenger having a health scare.

In some embodiments, the systems and methods of the present disclosure also provide an improvement to a computation system. For example, the systems and methods can receiving, at one or more computing devices, at least one signal indicative of a state of the passenger from one or more sensors of a chair in the aerial vehicle; determining, at the one or more computing devices, a health state of the passenger based on the at least one received signal; determining, at the one or more computing devices, if the aerial vehicle should land based on the determined health state of the passenger; and when a determination is made that the aerial vehicle should land based on the determined health state of the passenger: identifying, by the one or more computing devices, a nearest airport; and transmitting, by the one or more computing devices, a request to land to the identified airport.This can reduce computational resources needed to determine if an aerial vehicle should land to accommodate a passenger having a health scare.

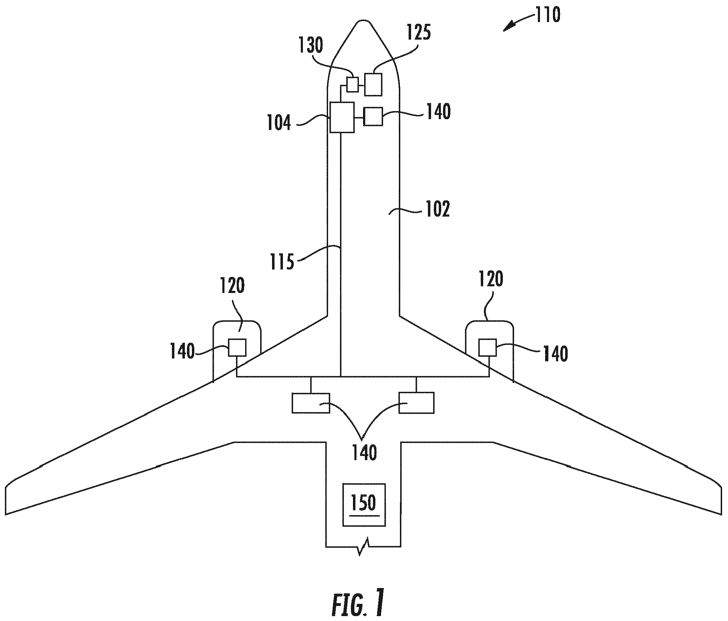

FIG. 1 depicts an example system for assessing a health of a passenger of an aerial vehicle according to example embodiments of the present disclosure. As shown, the system can include an aerial vehicle 102. The aerial vehicle 102 can include an onboard computing system 110. As shown in FIG. 1, the onboard computing system 110 can include one or more onboard computing device(s) 104 that can be associated with, for instance, an avionics system. The onboard computing device(s) 104 can be coupled to a variety of systems on the aerial vehicle 102 over a communications network 115. The communications network 115 can include a data bus or combination of wired and/or wireless communication links.

The onboard computing device(s) 104 can be in communication with a display system 125 including one or more display device(s) that can be configured to display or otherwise provide information generated or received by the system 110 to flight crew members of the aerial vehicle 102. The display system 125 can include a primary flight display, a multipurpose control display unit, or other suitable flight displays commonly included within a cockpit of the aerial vehicle 102.

The onboard computing device(s) 104 can also be in communication with a flight control computer 130. The flight control computer 130 can, among other things, automate the tasks of piloting and tracking the flight plan of the aerial vehicle 102. The flight control computer 130 can include or be associated with, any suitable number of individual microprocessors, power supplies, storage devices, interface cards, auto flight systems, flight management computers, and other standard components. The flight control computer 130 can include or cooperate with any number of software programs (e.g., flight management programs) or instructions designed to carry out the various methods, process tasks, calculations, and control/display functions necessary for operation of the aerial vehicle 102. The flight control computer 130 is illustrated as being separate from the onboard computing device(s) 104. Those of ordinary skill in the art, using the disclosures provided herein, will understand that the flight control computer 130 can also be included with or implemented by the onboard computing device(s) 104.

The onboard computing device(s) 104 can also be in communication with one or more aerial vehicle control system(s) 140. The aerial vehicle control system(s) 140 can be configured to perform various aerial vehicle operations and control various settings and parameters associated with the aerial vehicle 102. For instance, the aerial vehicle control system(s) 140 can be associated with one or more engine(s) 120 and/or other components of the aerial vehicle 102. The aerial vehicle control system(s) 140 can include, for instance, digital control systems, throttle systems, inertial reference systems, flight instrument systems, engine control systems, auxiliary power systems, fuel monitoring systems, engine vibration monitoring systems, communications systems, flap control systems, flight data acquisition systems, and other systems.

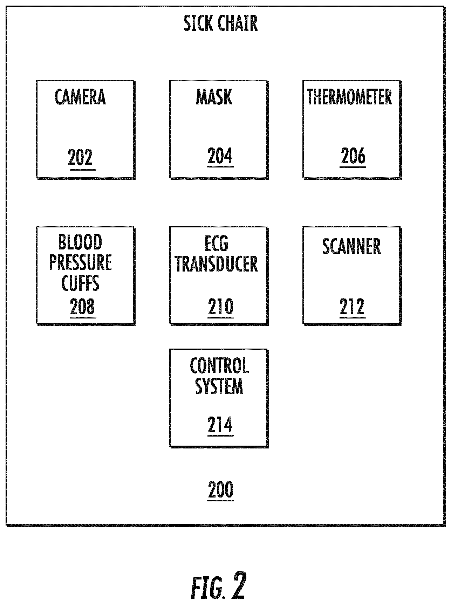

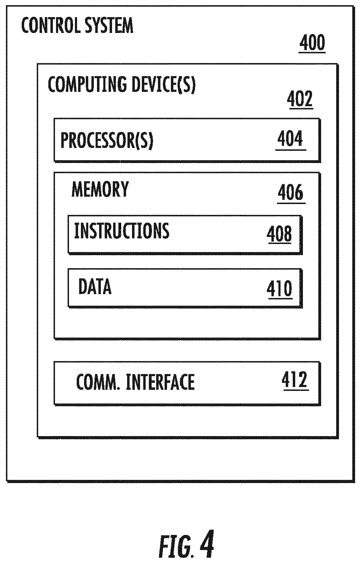

The aerial vehicle 102 can include a sick chair 150, described in more detail in reference to FIG. 2. The sick chair 150 can include or be in communication with a control system, such as the control system described in reference to FIG. 4. When a passenger is not feeling well, the passenger can be taken to the sick chair 150. Sensors in the sick chair 150 can gather information about the passenger. In an embodiment, passenger privacy can be preserved by not linking the sick chair 150 with any passenger identifying information, such as a customer ticket. In an embodiment, the gathered information can be compared with expected information by the control system. In an embodiment, the gathered information can be transmitted to a ground system, which can then transmit a recommendation back to the aerial vehicle 102. The gathered information can be used to determine if the aerial vehicle needs to land immediately for the passenger or not.

In another embodiment, the aerial vehicle 102 can be an aerial vehicle capable of autonomous flight used to transport one or more passengers from one location to another location. In such an embodiment, each passenger can reside in a sick chair 150 for the duration of the ride. If the sensors of one or more of the sick chairs indicates that one or more of the passengers are having a medical incident, a signal can be sent to a control system to take action. The control system can control components of the aerial vehicle so that the action can be taken. The action taken can include landing autonomously, providing or transmitting a notification, identifying a primary care provider, identifying a location associated with an emergency room and/or an identified primary care provider, adjusting a flight plan based on the identified location, the like, or any combination of the foregoing. When a determination is made that the aerial vehicle should not take action based on the determined health state of the passenger, the control system can maintain a current flight plan.

The numbers, locations, and/or orientations of the components of example aerial vehicle 102 are for purposes of illustration and discussion and are not intended to be limiting. Those of ordinary skill in the art, using the disclosures provided herein, shall understand that the numbers, locations, and/or orientations of the components of the aerial vehicle 102 can be adjusted without deviating from the scope of the present disclosure.

FIG. 2 depicts a block diagram of a sick chair 200 according to example embodiments of the present disclosure. The sick chair 200 can include one or more sensors. The one or more sensors can include one or more of: a mask 204, a camera 202, a thermometer 206, scales, stethoscope, blood pressure cuffs 208, a pulse oximeter with adult and pediatric probes, an echocardiogram (ECG) transducer 210, one or more tools for performing an electrocardiogram (EKG), tools for testing for one or more viruses, one or more bacteria, strep throat, pregnancy, etc. (such a scanner 212 for a litmus test), an ultrasonic transducer and/or machine, a blood glucose meter, and a spirometer. The sick chair 200 can include and/or be in communication with a control system 214. The control system 214 can be, for example, the control system 400 in FIG. 4. The control system 214 can be integrated into the sick chair. The control system 214 can be local to the aerial vehicle 102. For example, the control system 214 can be included in the onboard computing device(s) 104. The control system 214 can be remote from the aerial vehicle 102. For example, the control system 214 can be included in a ground system accessible through a satellite and/or radio network.

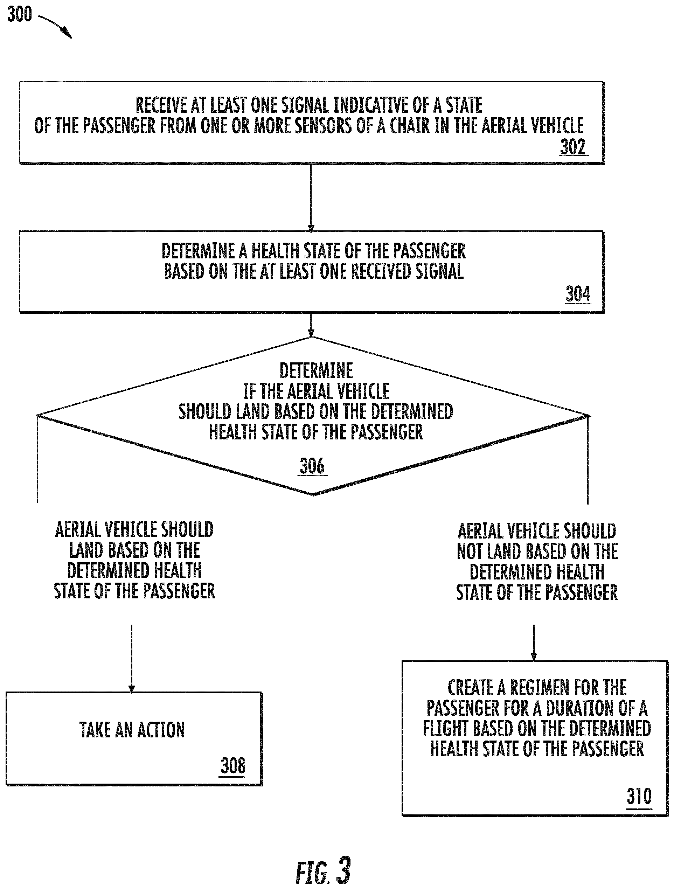

FIG. 3 depicts a flow diagram of an example method 300 for assessing a health of a passenger of an aerial vehicle. The method of FIG. 3 can be implemented using, for instance, the control system 400 of FIG. 4. FIG. 3 depicts steps performed in a particular order for purposes of illustration and discussion. Those of ordinary skill in the art, using the disclosures provided herein, will understand that various steps of any of the methods disclosed herein can be adapted, modified, rearranged, performed simultaneously or modified in various ways without deviating from the scope of the present disclosure.

At (302), at least one signal indicative of a state of the passenger can be received from one or more sensors of a chair in the aerial vehicle. For example, the control system 400 can receive at least one signal indicative of a state of the passenger from one or more sensors of a chair in the aerial vehicle. The one or more sensors can include one or more of: a mask, a camera, a thermometer, scales, stethoscope, blood pressure cuffs, a pulse oximeter with adult and pediatric probes, an echocardiogram (ECG) transducer, one or more tools for performing an electrocardiogram (EKG), tools for testing for one or more viruses, one or more bacteria, strep throat, pregnancy, etc. (such a scanner for a litmus test), an ultrasonic transducer and/or machine, a blood glucose meter, and a spirometer. In an embodiment, the at least one signal indicative of a state of the passenger can include one or more of: an eye color, an eye dilation, a skin color, a blood oxygen level, a glucose level, a body temperature, an alcohol level, a breathing level, a weight, a height, acoustics related to internal body parts (such as lungs, heart, etc.), a blood pressure, an image related to a body part (such as an ear, nose, throat, etc.), an ECG, an EKG, results of a test for one or more viruses, one or more bacteria, strep throat, pregnancy, etc. (such a scanner for a litmus test), and an ultrasonic imagine.

At (304), a health state of the passenger can be determined based on the at least one received signal. For example, the control system 400 can determine a health state of the passenger based on the at least one received signal. In an embodiment, the at least one received signal can be transmitted to a remote computing system. For example, the control system 400 can transmit the at least one received signal to a remote computing system. In an embodiment, the determined health state based on the at least one transmitted signal can be received from the remote computing system. For example, the control system 400 can receive the determined health state based on the at least one transmitted signal from the remote computing system. In an embodiment, the at least one received signal can be compared to at least one threshold value. For example, the control system 400 can compare the at least one received signal to at least one threshold value.

At (306), a determination can be made as to whether or not the aerial vehicle should land based on the determined health state of the passenger. For example, the control system 400 can determine if the aerial vehicle should land based on the determined health state of the passenger. When a determination is made that the aerial vehicle should land based on the determined health state of the passenger, the method 300 can move to (308). When a determination is made that the aerial vehicle should not land based on the determined health state of the passenger, the method 300 can move to (310).

At (308), an action can be taken. For example, the control system 400 can take an action. In an embodiment, a notification can be created. For example, the control system 400 can create a notification. In an embodiment, the notification can be caused to be created on an avionics system. For example, the control system 400 can cause the notification to be created on an avionics system. In an embodiment, the notification can be caused to be transmitted to a ground system. For example, the control system 400 can cause the notification to be transmitted to a ground system. In an embodiment, a flight plan can be adjusted. For example, the control system 400 can adjust a flight plan. In an embodiment, a nearest airport can be identified. For example, the control system 400 can identify a nearest airport. In an embodiment, a location associated with an emergency room or a primary care provider can be identified. For example, the control system 400 can identify a location associated with an emergency room or a primary care provider. The location associated with the emergency or the primary care provider can be an airport closest to the emergency room or the primary care provider. A request to land can be transmitted to the identified airport. For example, the control system 400 can transmit a request to land to the identified airport. In an embodiment, a communication indicating approval to land can be received. For example, the control system 400 can receive a communication indicating approval to land. In an embodiment, a flight plan can be adjusted in response to the received communication. For example, the control system 400 can adjust a flight plan in response to the received communication.

At (310), a regimen can be created for the passenger for a duration of a flight based on the determined health state of the passenger. For example, the control system 400 can create a regimen for the passenger for a duration of a flight based on the determined health state of the passenger.

FIG. 4 depicts a block diagram of an example control system 400 that can be used to implement methods and systems according to example embodiments of the present disclosure. The sick chair of FIG. 2, for example, can include and/or be in communication with the control system 400. As shown, the control system 400 can include one or more computing device(s) 402. The one or more computing device(s) 402 can include one or more processor(s) 404 and one or more memory device(s) 406. The one or more processor(s) 404 can include any suitable processing device, such as a microprocessor, microcontroller, integrated circuit, logic device, or other suitable processing device. The one or more memory device(s) 406 can include one or more computer-readable media, including, but not limited to, non-transitory computer-readable media, RAM, ROM, hard drives, flash drives, or other memory devices.

The one or more memory device(s) 406 can store information accessible by the one or more processor(s) 404, including computer-readable instructions 408 that can be executed by the one or more processor(s) 404. The instructions 408 can be any set of instructions that when executed by the one or more processor(s) 404, cause the one or more processor(s) 404 to perform operations. The instructions 408 can be software written in any suitable programming language or can be implemented in hardware. In some embodiments, the instructions 408 can be executed by the one or more processor(s) 404 to cause the one or more processor(s) 404 to perform operations, such as the operations for assessing a health of a passenger of an aerial vehicle, as described with reference to FIG. 3.

The memory device(s) 406 can further store data 410 that can be accessed by the one or more processor(s) 404. For example, the data 410 can include any data used for assessing a health of a passenger of an aerial vehicle, as described herein. The data 410 can include one or more table(s), function(s), algorithm(s), model(s), equation(s), etc. for assessing a health of a passenger of an aerial vehicle according to example embodiments of the present disclosure.

The one or more computing device(s) 402 can also include a communication interface 412 used to communicate, for example, with the other components of system. The communication interface 412 can include any suitable components for interfacing with one or more network(s), including for example, transmitters, receivers, ports, controllers, antennas, or other suitable components.

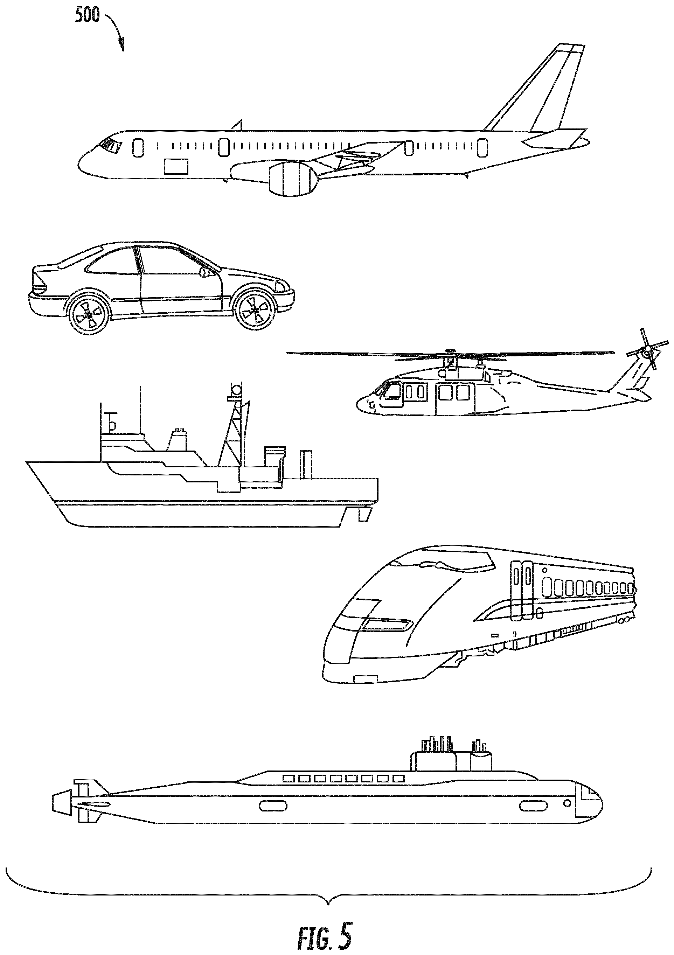

Referring now to FIG. 5, example vehicles 500 according to example embodiments of the present disclosure are depicted. The systems and methods of the present disclosure can be implemented on an aerial vehicle, helicopter, automobile, boat, submarine, train, and/or any other suitable vehicles. While the present disclosure is described herein with reference to an aerial vehicle implementation, this is intended only to serve as an example and not to be limiting. One of ordinary skill in the art would understand that the systems and methods of the present disclosure can be implemented on other vehicles without deviating from the scope of the present disclosure.

The technology discussed herein makes reference to computer-based systems and actions taken by and information sent to and from computer-based systems. One of ordinary skill in the art will recognize that the inherent flexibility of computer-based systems allows for a great variety of possible configurations, combinations, and divisions of tasks and functionality between and among components. For instance, processes discussed herein can be implemented using a single computing device or multiple computing devices working in combination. Databases, memory, instructions, and applications can be implemented on a single system or distributed across multiple systems. Distributed components can operate sequentially or in parallel.

Although specific features of various embodiments may be shown in some drawings and not in others, this is for convenience only. In accordance with the principles of the present disclosure, any feature of a drawing may be referenced and/or claimed in combination with any feature of any other drawing.

This written description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they include structural elements that do not differ from the literal language of the claims, or if they include equivalent structural elements with insubstantial differences from the literal languages of the claims.

Various aspects and embodiments of the present invention are defined by the following numbered clauses:
1. A system for assessing a health of a passenger of an aerial vehicle comprising:
   a memory device; and
   one or more processors configured to:
      receive at least one signal indicative of a state of the passenger from one or more sensors of a chair in the aerial vehicle;
      determine a health state of the passenger based on the at least one received signal;
      determine if the aerial vehicle should land based on the determined health state of the passenger; and
      when a determination is made that the aerial vehicle should land based on the determined health state of the passenger, adjust a flight plan.
2. The system of clause 1, wherein the one or more processors are further configured to:
   transmit the at least one received signal to a remote computing system; and
   receive the determined health state based on the at least one transmitted signal from the remote computing system.
3. The system of any preceding clause, wherein the one or more processors are further configured to compare the at least one received signal to at least one threshold value.
4. The system of any preceding clause, wherein the one or more sensors comprise one or more of: a mask, a camera, a thermometer, scales, stethoscope, blood pressure cuffs, a pulse oximeter with adult and pediatric probes, an echocardiogram transducer, one or more tools for performing an electrocardiogram, tools for testing for one or more viruses, one or more bacteria, strep throat, pregnancy, an ultrasonic transducer, an ultrasonic machine, a blood glucose meter, a spirometer, or a tool for testing one or more viruses, one or more bacteria, strep throat, or pregnancy.
5. The system of any preceding clause, wherein the at least one signal indicative of a state of the passenger comprises one or more of: an eye color, an eye dilation, a skin color, a blood oxygen level, a glucose level, a body temperature, an alcohol level, a breathing level, a weight, a height, acoustics related to internal body parts, a blood pressure, an image related to a body part, an echocardiogram, an electrocardiogram, an ultrasonic imagine, or results of a test for one or more viruses, one or more bacteria, strep throat, or pregnancy.
6. The system of any preceding clause, wherein when a determination is made that the aerial vehicle should not land based on the determined health state of the passenger, the one or more processors are further configured to create a regimen for the passenger for a duration of a flight based on the determined health state of the passenger.
7. The system of any preceding clause, wherein the one or more processors are further configured to:
   cause the notification to be created on an avionics system; and
   cause the notification to be transmitted to a ground system.
8. A method for assessing a health of a passenger of an aerial vehicle comprising:
   receiving, at one or more computing devices, at least one signal indicative of a state of the passenger from one or more sensors of a chair in the aerial vehicle;
   determining, at the one or more computing devices, a health state of the passenger based on the at least one received signal;
   determining, at the one or more computing devices, if the aerial vehicle should land based on the determined health state of the passenger; and
   when a determination is made that the aerial vehicle should land based on the determined health state of the passenger:
      identifying, by the one or more computing devices, a nearest airport; and
      transmitting, by the one or more computing devices, a request to land to the identified airport.
9. The method of any preceding clause, further comprising:
   receiving, by the one or more computing devices, a communication indicating approval to land; and
   adjusting, by the one or more computing devices, a flight plan in response to the received communication.
10. The method of any preceding clause, wherein determining, at the one or more computing devices, a health state of the passenger based on the at least one received signal further comprises:
   transmitting, from the one or more computing devices, the at least one received signal to a remote computing system; and
   receiving, at the one or more computing devices, the determined health state based on the at least one transmitted signal from the remote computing system.
11. The method of any preceding clause, wherein determining, at the one or more computing devices, a health state of the passenger based on the at least one received signal further comprises comparing the at least one received signal to at least one threshold value.
12. The method of any preceding clause, wherein the one or more sensors comprise one or more of: a mask, a camera, a thermometer, scales, stethoscope, blood pressure cuffs, a pulse oximeter with adult and pediatric probes, an echocardiogram transducer, one or more tools for performing an electrocardiogram, tools for testing for one or more viruses, one or more bacteria, strep throat, pregnancy, an ultrasonic transducer, an ultrasonic machine, a blood glucose meter, a spirometer, or a tool for testing one or more viruses, one or more bacteria, strep throat, or pregnancy.
13. The method of any preceding clause, wherein the at least one signal indicative of a state of the passenger comprises one or more of: an eye color, an eye dilation, a skin color, a blood oxygen level, a glucose level, a body temperature, an alcohol level, a breathing level, a weight, a height, acoustics related to internal body parts, a blood pressure, an image related to a body part, an echocardiogram, an electrocardiogram, an ultrasonic imagine, or results of a test for one or more viruses, one or more bacteria, strep throat, or pregnancy.
14. The method of any preceding clause, wherein when a determination is made that the aerial vehicle should not land based on the determined health state of the passenger, creating, by the one or more computing devices, a regimen for the passenger for a duration of a flight based on the determined health state of the passenger.
15. The method of any preceding clause, wherein creating, by the one or more computing devices, a notification further comprises:
   causing, by the one or more computing devices, the notification to be created on an avionics system; and
   causing, by the one or more computing devices, the notification to be transmitted to a ground system.
16. An aerial vehicle capable of autonomous flight comprising:
   a memory device; and
   one or more processors configured to:
      receive at least one signal indicative of a state of the passenger from one or more sensors of a chair in the aerial vehicle;
      determine a health state of the passenger based on the at least one received signal;
      determine if the aerial vehicle should take action based on the determined health state of the passenger; and
      when a determination is made that the aerial vehicle should take action based on the determined health state of the passenger, send a signal to a control system to take an action.
17. The aerial vehicle of any preceding clause, wherein the signal causes the control system to:
   transmit a notification of an emergency; and
   land the aerial vehicle autonomously.
18. The aerial vehicle of any preceding clause, wherein the signal causes the control system to:
   identify a location associated with an emergency room; and
   adjust a flight plan based on the identified location.
19. The aerial vehicle of any preceding clause, wherein the signal causes the control system to:
   identify a primary care provider associated with the passenger;
   identify a location associated with the identified primary care provider; and
   adjust destination flight plan based on the identified location.
20. The aerial vehicle of any preceding clause, wherein when a determination is made that the aerial vehicle should not take action based on the determined health state of the passenger, the one or more processors are further configured to maintain a current flight plan.

## Claims

1. A system for assessing a health of a passenger of an aerial vehicle (102) comprising:
a memory device (406); and
one or more processors (404) configured to:
receive at least one signal indicative of a state of the passenger from one or more sensors of a chair in the aerial vehicle (102);
determine a health state of the passenger based on the at least one received signal;
determine if the aerial vehicle (102) should land based on the determined health state of the passenger; and
when a determination is made that the aerial vehicle (102) should land based on the determined health state of the passenger, adjust a flight plan.

2. The system of claim 1, wherein the one or more processors (404) are further configured to:
transmit the at least one received signal to a remote computing system (110); and
receive the determined health state based on the at least one transmitted signal from the remote computing system (110).

3. The system of either of claim 1 or 2, wherein the one or more processors (404) are further configured to compare the at least one received signal to at least one threshold value.

4. The system of any preceding claim, wherein the one or more sensors comprise one or more of: a mask (204), a camera (202), a thermometer (206), scales, stethoscope, blood pressure cuffs (208), a pulse oximeter with adult and pediatric probes, an echocardiogram transducer (210), one or more tools for performing an electrocardiogram, tools for testing for one or more viruses, one or more bacteria, strep throat, pregnancy, an ultrasonic transducer, an ultrasonic machine, a blood glucose meter, a spirometer, or a tool for testing one or more viruses, one or more bacteria, strep throat, or pregnancy.

5. The system of any preceding claim, wherein the at least one signal indicative of a state of the passenger comprises one or more of: an eye color, an eye dilation, a skin color, a blood oxygen level, a glucose level, a body temperature, an alcohol level, a breathing level, a weight, a height, acoustics related to internal body parts, a blood pressure, an image related to a body part, an echocardiogram, an electrocardiogram, an ultrasonic imagine, or results of a test for one or more viruses, one or more bacteria, strep throat, or pregnancy.

6. The system of any preceding claim, wherein when a determination is made that the aerial vehicle (102) should not land based on the determined health state of the passenger, the one or more processors (404) are further configured to create a regimen for the passenger for a duration of a flight based on the determined health state of the passenger.

7. The system of any preceding claim, wherein the one or more processors (404) are further configured to:
cause the notification to be created on an avionics system; and
cause the notification to be transmitted to a ground system.

8. A method for assessing a health of a passenger of an aerial vehicle (102) comprising:
receiving, at one or more computing devices (402), at least one signal indicative of a state of the passenger from one or more sensors of a chair in the aerial vehicle (102);
determining, at the one or more computing devices (402), a health state of the passenger based on the at least one received signal;
determining, at the one or more computing devices (402), if the aerial vehicle (102) should land based on the determined health state of the passenger; and
when a determination is made that the aerial vehicle (102) should land based on the determined health state of the passenger:
identifying, by the one or more computing devices (402), a nearest airport; and
transmitting, by the one or more computing devices (402), a request to land to the identified airport.

9. The method of claim 8, further comprising:
receiving, by the one or more computing devices (402), a communication indicating approval to land; and
adjusting, by the one or more computing devices (402), a flight plan in response to the received communication.

10. The method of either of claim 8 or 9, wherein determining, at the one or more computing devices (402), a health state of the passenger based on the at least one received signal further comprises:
transmitting, from the one or more computing devices (402), the at least one received signal to a remote computing system (110); and
receiving, at the one or more computing devices (402), the determined health state based on the at least one transmitted signal from the remote computing system (110).

11. The method of any of claims 8 to 10, wherein determining, at the one or more computing devices (402), a health state of the passenger based on the at least one received signal further comprises comparing the at least one received signal to at least one threshold value.

12. The method of any of claims 8 to 11, wherein the one or more sensors comprise one or more of: a mask (204), a camera (202), a thermometer (206), scales, stethoscope, blood pressure cuffs (208), a pulse oximeter with adult and pediatric probes, an echocardiogram transducer (210), one or more tools for performing an electrocardiogram, tools for testing for one or more viruses, one or more bacteria, strep throat, pregnancy, an ultrasonic transducer, an ultrasonic machine, a blood glucose meter, a spirometer, or a tool for testing one or more viruses, one or more bacteria, strep throat, or pregnancy.

13. The method of any of claims 8 to 12, wherein the at least one signal indicative of a state of the passenger comprises one or more of: an eye color, an eye dilation, a skin color, a blood oxygen level, a glucose level, a body temperature, an alcohol level, a breathing level, a weight, a height, acoustics related to internal body parts, a blood pressure, an image related to a body part, an echocardiogram, an electrocardiogram, an ultrasonic imagine, or results of a test for one or more viruses, one or more bacteria, strep throat, or pregnancy.

14. The method of any of claims 8 to 13, wherein when a determination is made that the aerial vehicle (102) should not land based on the determined health state of the passenger, creating, by the one or more computing devices (402), a regimen for the passenger for a duration of a flight based on the determined health state of the passenger.

15. The method of any of claims 8 to 14, wherein creating, by the one or more computing devices (402), a notification further comprises:
causing, by the one or more computing devices (402), the notification to be created on an avionics system; and
causing, by the one or more computing devices (402), the notification to be transmitted to a ground system.
